# EUROPÄISCHE PATENTANMELDUNG

(11) **EP 2 146 296 A2**
(43) Veröffentlichungstag der Anmeldung: **20.01.2010**
(21) Anmeldenummer: 09163441.0
(22) Anmeldetag: 23.06.2009
(51) Int. Cl.: G06F 19/00

(54) **Medizinisches Implantat mit mindestens zwei Datenkommunikationskanälen**

(30) Priorität: 17.07.2008 DE 102008040502
(71) Anmelder: Biotronik CRM Patent AG, 6341 Baar (CH)
(72) Erfinder: Dörr, Thomas, 12437 Berlin (DE)
(74) Vertreter: Lindner-Vogt, Karin L.

(57) **Zusammenfassung**

Die Erfindung betrifft ein medizinisches Implantat, ein Therapiesystem mit einem medizinischen Implantat, ein Verfahren und ein Programmiergerät für ein medizinisches Implantat, bei dem das medizinische Implantat wenigstens eine Schnittstelle zum bidirektionalen drahtlosen Datenaustausch, einen Datenspeicher und eine mit der Schnittstelle und dem Datenspeicher verbundenen Steuerung aufweist. Die Steuerung ist in Verbindung mit der Schnittstelle ausgebildet, einen drahtlosen Datenaustausch mit wenigstens zwei unterschiedlichen externen Geräten durchzuführen, so dass sich wenigstens zwei verschiedene Datenkommunikationskanäle ergeben, von denen ein erster der Datenkommunikation mit einem Programmiergerät zugeordnet ist und ein zweiter Datenkommunikationskanal der Datenkommunikation mit einem zentralen Servicecenter zugeordnet ist. Das medizinische Implantat ist weiter ausgebildet, über den zweiten Datenkommunikationskanal Steuerbefehle und/oder Nachrichten zu empfangen, während der zweite Datenkommunikationskanal aktiv ist, empfangene Steuerbefehle und/oder Nachrichten, die für ein Programmiergerät bestimmt sind, zu erkennen, erforderlichenfalls in dem Speicher zwischenzuspeichern und über den ersten Datenkommunikationskanal an ein Programmiergerät weiterzuleiten, während der erste Datenkommunikationskanal aktiv ist.

## Beschreibung

Die Erfindung betrifft ein medizinisches Implantat, also ein implantierbares medizinisches Gerät, wie beispielsweise einen Herzschrittmacher, Cardioverter/Defibrillator oder dergleichen.

Derartige medizinische Implantate besitzen heutzutage häufig wenigstens eine Datenkommunikationsschnittstelle für einen drahtlosen Datenaustausch mit wenigstens einem externen Gerät, beispielsweise einem Programmiergerät oder einem so genannten Patientengerät, das als Relaisstation für einen Datenaustausch mit einem zentralen Servicecenter dient.

Die Programmierung und Abfrage eines derartigen Implantats mit Hilfe eines Programmiergerätes ist hinlänglich bekannt. Beispielsweise sind solche medizinischen Implantate, wie Herzschrittmacher und/oder Cardioverter/Defibrillatoren häufig zum einen in der Lage, während des Betriebs physiologische Daten, beispielsweise intrakardiale Elektrokardiogramme oder ähnliches, aufzuzeichnen und im Falle der Abfrage durch ein Programmiergerät diese Daten zu dem Programmiergerät zu übertragen. Umgekehrt ist es möglich, mit Hilfe des Programmiergerätes beispielsweise eine Umprogrammierung der Steuerung des medizinischen Implantates vorzunehmen. Derartiges geschieht üblicherweise, wenn ein das medizinische Implantat tragender Patient den betreuenden Arzt aufsucht. Die geringe Reichweite der drahtlosen Datenübertragung zwischen medizinischem Implantat und externem Programmiergerät setzt dabei eine große Nähe zwischen Implantat und Programmiergerät voraus, die zur Folge hat, dass bei einer derartigen Nachsorgeuntersuchung durch einen Arzt auch Patient und Arzt einander gegenübertreten. Der drahtlose Datenaustausch zwischen medizinischem Implantat und Programmiergerät erfolgt dabei über einen ersten, dem Programmiergerät zugeordneten Datenkommunikationskanal des medizinischen Implantats.

Neben diesem direkten Datenaustausch zwischen medizinischem Implantat und Programmiergerät sind heutzutage viele medizinische Implantate dazu in der Lage, über einen zweiten Datenkommunikationskanal Daten drahtlos mit einem externen Gerät in Form eines Patientengerätes auszutauschen, welches seinerseits wiederum mit einem zentralen Servicecenter verbunden ist. Die Datenkommunikationsverbindung zwischen Patientengerät und zentralem Servicecenter kann dabei drahtgebunden sein und beispielsweise auch über das Internet erfolgen. Auch über den zweiten Datenkommunikationskanal können vom medizinischen Implantat gewonnene Daten, beispielsweise physiologische Daten oder Betriebsdaten, mit dem zentralen Servicecenter ausgetauscht werden. Umgekehrt können Programmier- oder Steuerbefehle vom zentralen Servicecenter zum medizinischen Implantat über den zweiten Datenkommunikationskanal übertragen werden.

Üblicherweise ist für den ersten Datenkommunikationskanal und den zweiten Datenkommunikationskanal jeweils eine eigene Schnittstelle vorgesehen, so dass ein derartiges medizinisches Implantat zwei Datenkommunikationsschnittstellen aufweist. Es ist aber auch möglich, für die Datenkommunikation über den ersten Datenkommunikationskanal und den zweiten Datenkommunikationskanal dieselbe Schnittstelle vorzusehen.

Aufgabe der Erfindung ist es, ein derartiges System, bestehend aus medizinischem Implantat, Programmiergerät und zentralem Servicecenter, zu verbessern.

Erfindungsgemäß wird diese Aufgabe durch ein medizinisches Implantat mit wenigstens einer Schnittstelle zum bidirektionalen drahtlosen Datenaustausch, einen Datenspeicher und einer mit der Schnittstelle und dem Datenspeicher verbunden Steuerung gelöst, wobei die Steuerung in Verbindung mit der Schnittstelle ausgebildet ist, einen drahtlosen Datenaustausch mit wenigstens zwei unterschiedlichen externen Geräten durchzuführen, so dass sich wenigstens zwei verschiedene Datenkommunikationskanäle ergeben, von denen ein erster Datenkommunikationskanal der Datenkommunikation mit einem Programmiergerät zugeordnet ist und ein zweiter Datenkommunikationskanal der Datenkommunikation mit einem zentralen Servicecenter. Erfindungsgemäß ist das medizinische Implantat weiter ausgebildet, über den zweiten Datenkommunikationskanal Steuerbefehle und/oder Nachrichten, die für ein Programmiergerät bestimmt sind, zu empfangen, während der zweite Datenkommunikationskanal aktiv ist, etwa empfangene Steuerbefehle und/oder Nachrichten erforderlichenfalls in dem Speicher zwischenzuspeichern und über den ersten Datenkommunikationskanal an ein Programmiergerät weiterzuleiten, wenn der erste Datenkommunikationskanal aktiv ist.

Dabei kann die Schnittstelle als gemeinsame Datenkommunikationsschnittstelle für beide Datenkommunikationskanäle dienen oder es kann für jeden Datenkommunikationskanal eine eigene Schnittstelle vorgesehen sein.

Die Steuerung des medizinischen Implantates ist so ausgebildet, dass sie über den zweiten Datenkommunikationskanal empfangene Steuerbefehle und/oder Nachrichten, die an das Programmiergerät gerichtet sind, selbsttätig erkennt und entsprechend für eine ggf. erforderliche Zwischenspeicherung sowie die automatische Übertragung vom medizinischen Implantat zum Programmiergerät sorgt, sobald der erste Datenkommunikationskanal aktiv ist.

Mit einem derartigen medizinischen Implantat ist es möglich, einem jeweils betreuenden Arzt Nachrichten seitens des zentralen Servicecenters zu übermitteln, die dem jeweils betreuenden Arzt dann über sein jeweiliges Programmiergerät angezeigt werden. Darüber hinaus kann auch - je nach Art des Programmiergerätes - ein Programmiergerät dazu veranlasst werden, selbsttätig beispielsweise bestimmte Daten aus dem Implantat abzufragen. Das heißt, es kann eine indirekte Programmierung des Programmiergerätes durch das zentrale Servicecenter erfolgen, wobei das medizinische Implantat (ggf. ein zugehöriges Patientengerät) als Relaisstationen dienen.

Auf diese Weise können einem einen jeweiligen Patienten betreuenden Arzt gezielt Nachrichten übermittelt werden, ohne dass der Arzt von sich aus aktiv Kontakt zu einem zentralen Servicecenter aufnehmen muss.

Damit ist es möglich alle wichtigen Informationen, die in einem zentralen Servicecenter bezogen auf einen Patienten mit einem elektronischen Implantat verfügbar sind auch an den nachsorgenden Arzt zuverlässig weiterzuleiten, ohne dass dieser einen Zugang zu dem zentralen Servicecenter haben muss. Ferner ist es möglich, automatisch Funktionen des Programmiergerätes auf Basis von Informationen, die in das zentrale Servicecenter eingestellt werden, zu steuern, wie z.B. das Veranlassen eine Firmware-Up-Date bei einem definierten Seriennummernkreis im Rahmen der nächsten Nachsorge.

Ebenso können wichtige Nachrichten des Herstellers elektronischer Implantate (z.B. Safety Advisories) an den Nachsorgearzt übermittelt werden, ohne den Nachsorgearzt identifizieren zu müssen.

Gemäß einer bevorzugten Ausführungsvariante des Implantats besitzt dieses wenigstens zwei Schnittstellen, von denen eine erste dem ersten Datenkommunikationskanal für einen bidirektionalen, drahtlosen Datenaustausch mit einem Programmiergerät zugeordnet ist und die zweite Schnittstelle dem zweiten Datenkommunikationskanal für einen bidirektionalen, drahtlosen Datenaustausch mit einem zentralen Servicecenter.

Ein erfindungsgemäßes Programmiergerät zur drahtlosen Datenabfrage und Programmierung eines medizinischen Implantats besitzt eine Schnittstelle zum bidirektionalen, drahtlosen Datenaustausch mit einem medizinischen Implantat, einen Datenspeicher, einer Anzeige und eine mit der Schnittstelle des Programmiergeräts, der Anzeige und dem Datenspeicher verbundene Steuerung. Diese Steuerung ist ausgebildet, über die Schnittstelle empfangenen Datenpakete wenigstens derart zu unterscheiden und zu verarbeiten, dass Datenpakete die eine Textnachricht enthalten zum Anzeigen der Textnachricht auf der Anzeige des Programmiergerätes führen und Datenpakete, die Steuer- oder Programmierbefehle enthalten zum Ausführen dieser Steuer- oder Programmierbefehle.

Das Programmiergerät kann ausgebildet sein, über eine weitere Schnittstelle ebenfalls Informationen mit dem zentralen Servicecenter auszutauschen. So kann der Arzt beispielsweise informiert werden, wenn die vom medizinischen Implantat über den zweiten Datenkommunikationskanal an das Servicecenter übermittelten Daten Auffälligkeiten aufweisen, die die besondere Aufmerksamkeit des Arztes erfordern. Ebenso können über diese weitere Schnittstelle Aktualisierungen der Software des Programmiergerätes erfolgen. Weist das Programmiergerät die oben genannte weitere Schnittstelle zum zentralen Servicecenter nicht auf, beziehungsweise ist die Kommunikation zwischen Programmiergerät und zentralen Servicecenter aus verschiedenen Gründen nicht möglich, können diese Informationen in bekannten Systemen nicht übermittelt werden.

Vorzugsweise ist das Programmiergerät weiter ausgebildet, nach Empfang eines von einem zentralen Servicecenter generierten Datenpakets eine an das jeweilige zentrale Servicecenter gerichtete Empfangsbestätigung zu generieren und über den ersten Datenkommunikationskanal an ein medizinisches Implantat zu übertragen.

In diesem Zusammenhang ist der Begriff Datenpaket so zu verstehen, das ein jeweiliges die hierin erwähnten Datenpakete auch in Form einer Vielzahl von Teildatenpaketen übertragen werden kann. Mit Datenpaket ist somit nicht nur ein einzelnes Teildatenpaket gemeint, wie es je nach Übertragungsprotokoll bei der Datenübertragung vorkommt.

Ein weiterer Aspekt der vorliegenden Erfindung besteht auch in einem medizinischen Therapiesystem mit einem Implantat der zuvor beschriebenen Art sowie einem zentralen Servicecenter, welches zeitweise über einen Datenkommunikationskanal (dies ist der zweite Datenkommunikationskanal) mit dem medizinischen Implantat zum Datenaustausch verbunden ist, wobei das zentrale Servicecenter ausgebildet, an ein mit dem medizinischen Implantat zu verbindendes Programmiergerät gerichtete Datenpakete mit Textnachrichten und/oder Steuer- und Programmierbefehlen zu generieren und an das medizinische Implantat zu übertragen, wenn der (zweite) Datenkommunikationskanal aktiv ist. In diesem Sinne ist auch ein zentrales Servicecenter, das ausgebildet ist, an ein mit dem medizinischen Implantat zu verbindendes Programmiergerät gerichtete Datenpakete mit Textnachrichten und/oder Steuer- und Programmierbefehlen zu generieren, ein eigenständiger Erfindungsaspekt. Vorzugsweise weist das zentrale Servicecenter dabei eine Schnittstelle zur Eingabe von Textnachrichten auf, die für ein Programmiergerät bestimmt sind. Auf diese Weise kann ein mit dem zentralen Servicecenter verbundener Homemonitoring-Arzt Nachrichten an einen für die Nachsorge eines Implantatspatienten zuständigen Nachsorgearzt generieren und sich sicher sein, dass dieser Nachsorgearzt die Nachricht zum richtigen Zeitpunkt, nämlich dann, wenn er mittels eines Programmiergerätes einen Kontakt zu einem jeweiligen Implantat herstellt, empfängt.

Insgesamt ergibt sich somit ein System, das mindestens ein elektronisches Implantat, ein zentrales Servicecenter und ein Programmiergerät aufweist, wobei das Implantat vorzugsweise über einen zweiten Datenkommunikationskanal derart mit dem zentralen Servicecenter verbunden ist, dass es regelmäßig Informationen an das zentrale Servicecenter über eine Relaisstation (also ein Patientengerät bzw. ein externes Geräte) sendet und umgekehrt Informationen von dem zentralen Servicecenter empfangen und speichern kann. Solche im Implantat gespeicherte Informationen können dann wenn sie an das Programmiergerät gerichtet sind, von dem bei der Nachsorge eingesetzten Programmiergerätes ausgelesen und zur Anzeige oder zur Steuerung von automatischen Reaktionen des Programmiergerätes genutzt werden.

Es ergeben sich die folgenden, vorteilhaften Varianten eines solchen Systems bzw. seiner Systemskomponenten (medizinisches Implantat, Programmiergerät oder zentrales Servicecenter):
- (a): Die vom zentralen Servicecenter an das Implantat übermittelte Information, die dann mit dem Programmiergerät des Nachsorgearztes abgefragt und verarbeitet wird, wird manuell in das zentrale Servicecenter eingegeben.
- (b): Die vom zentralen Servicecenter an das Implantat übermittelte Information, die dann mit dem Programmiergerät des Nachsorgearztes abgefragt und verarbeitet wird, wird automatisch im zentralen Servicecenter im Ergebnis einer Datenanalyse generiert.
- (c): Die vom zentralen Servicecenter an das Implantat übermittelte Information, die dann mit dem Programmiergerät des Nachsorgearztes abgefragt und verarbeitet wird, wird mittels einer Datenübertragungsschnittstelle der Herstellers der Implantate zu dem zentralen Servicecenter übergeben (z.B. Seriennummernlisten mit zugeordneten Firmwareversionen).
- (d): Die vom zentralen Servicecenter an das Implantat übertragene Information wird bei Abfrage durch das Programmiergerät zur Steuerung des Programmiergerätes verwendet.
- (e): Die vom zentralen Servicecenter an das Implantat übertragene Information wird bei Abfrage durch das Programmiergerät zur Steuerung eines Firmware-Update der Implantatsfirmware verwendet.
- (f): Die vom zentralen Servicecenter an das Implantat übertragene Information wird bei Abfrage durch das Programmiergerät zur automatischen Umprogrammierung mindestens eines Parameters des elektronischen Implantates verwendet.
- (g): Die Abfrage der durch das zentrale Servicecenter in das elektronische Implantat übertragene Information durch das Programmiergerät wird durch das Programmiergerät bestätigt und diese Bestätigung wird dann mit der periodischen Nachricht an das zentrale Servicecenter übermittelt.

Die Erfindung soll nun anhand von Ausführungsbeispielen mit Bezug auf die Figuren näher erläutert werden. Von den Figuren zeigt
- Fig. 1:: Ein medizinisches Therapiesystem mit einem erfindungsgemäßen Implantat, einem erfindungsgemäßen Programmiergerät und einem erfindungsgemäßen zentralen Servicecenter;
- Fig. 2:: eine schematische Darstellung eines erfindungsgemäßen medizinischen Implantats;
- Fig. 3:: eine schematische Darstellung eines erfindungsgemäßen Programmiergerätes;
- Fig. 4:: eine schematische Darstellung eines erfindungsgemäßen Servicecenters;
- Fig. 5:: das System aus Fig. 1 im Falle einer Information eines Nachsorgearztes durch einen Home Monitoring Arzt;
- Fig. 6:: das System aus Fig. 1 im Falle einer automatischen Information eines Nachsorgearztes durch eine automatische Analyse des zentralen Servicecenters;
- Fig. 7:: das System aus Fig. 1 im Falle der Verteilung einer "Safety Advisory" durch den Hersteller des Implantats;
- Fig. 8:: den Ablauf im Falle eines Firmware-Updates; und
- Fig. 9:: den Ablauf im Falle der Steuerung einer Programmiergerätkomponente durch einen Steuerbefehl seitens des zentralen Servicecenters.

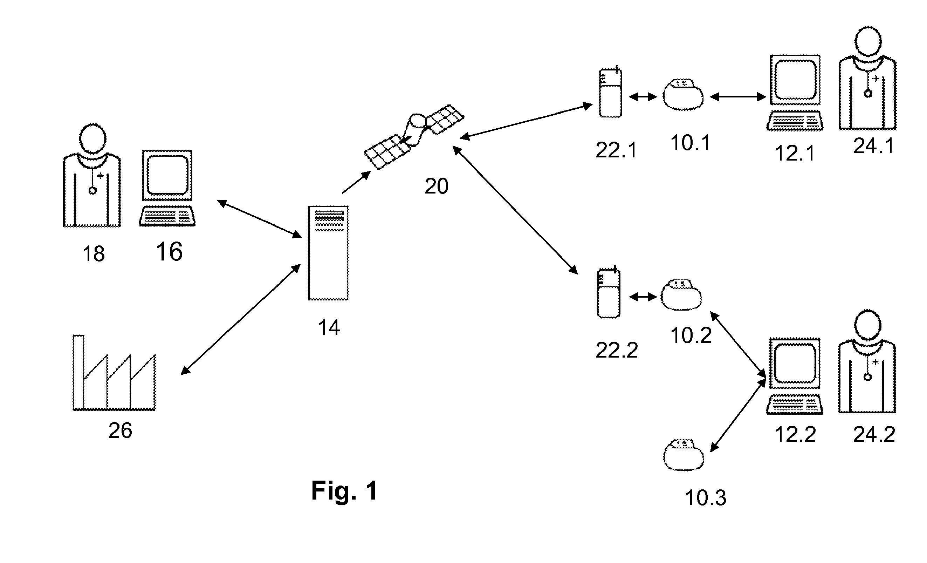

In Fig. 1 ist die Übersicht über ein erfindungsgemäßes Therapiesystem dargestellt, das ein erfindungsgemäßes medizinisches Implantat 10, ein diesem zugeordnetes Programmiergerät 12 und ein zentrales Servicecenter mit einem Server 14 umfasst. Die Darstellung enthält zum besseren Verständnis mehrere medizinische Implantate 10, dargestellt als 10.1, 10.2, 10.3, des weiteren mehrere Programmiergeräte 12, dargestellt als 12.1 und 12.2, darüber hinaus mehrere Patientenindividuelle Relaisstationen 22, dargestellt als 22.1 und 22.2, sowie mehrere Nachsorgeärzte 24, dargestellt als 24.1 und 24.2. Für die Funktionsweise des erfindungsgemäßen Therapiesystems ist jeweils eines der oben genannten mehrfach vorhandenen Bestandteile ausreichend.

Der Server 14 ist mit einem Terminal 16 verbunden, über das ein Arzt 18 Zugriff auf den Server 14 zur Fernüberwachung von Implantaten 10 hat. Der Server 14 kommuniziert mittels einer Kommunikationsverbindung 20 mit mehreren patientenindividuellen Relaisstationen 22, die beispielsweise die bereits erwähnten Patientengeräte sind. Diese wiederum kommunizieren mit einem jeweiligen elektronischen Implantat 10 eines Patienten. Die Datenverbindungsstrecke zwischen dem Server 14 und einem jeweiligen Implantat 10 über die jeweiligen Relaisstationen 22 stellen dabei einen zweiten Datenkommunikationskanal dar, während eine Datenübertragungsstrecke zwischen einem jeweiligen Implantat 10 und einem zugeordneten Programmiergerät 12 einen ersten Datenkommunikationskanal darstellt. Beide Datenkommunikationskanäle sind in der Regel nicht ständig, sondern nur zeitweise aktiv, schon alleine um Sendeenergie seitens dem Implantats 10 zu sparen.

In periodischen Abständen werden die elektronischen Implantate 10 von Nachsorgeärzten 24 mittels eines jeweiligen Programmiergerätes 12 abgefragt und bei Bedarf umprogrammiert. Dies geschieht über den ersten Datenkommunikationskanal. Hierbei werden auch solche elektronischen Implantate 10 regelmäßig abgefragt, die keine Verbindung zum Server 14 des zentralen Servicecenters haben. Ein solches elektronisches Implantat ist als 10.3 dargestellt. Gemäß einer bevorzugten Ausführungsvariante des Gesamtsystems haben auch die Hersteller 26 des jeweiligen elektronischen Implantats 10 einen Zugriff auf den Server 14 des zentralen Servicecenters, um so eine Fernüberwachung der elektronischen Implantate 10 durch den jeweiligen Hersteller 26 zu erlauben.

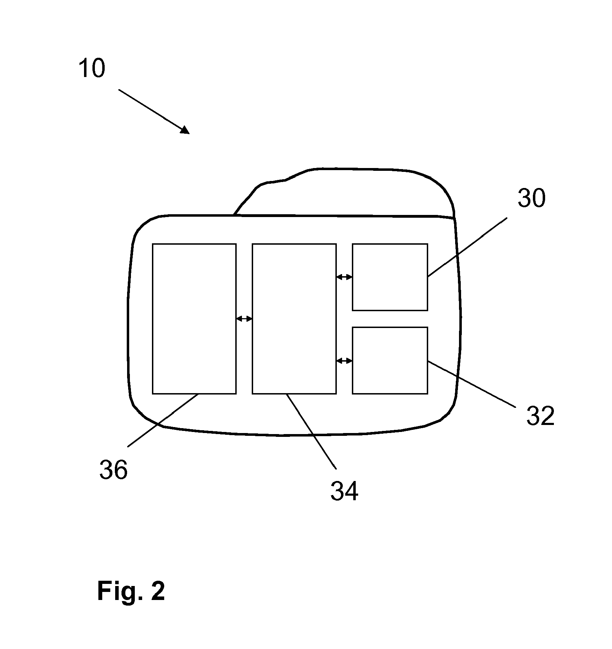

Fig. 2 zeigt, dass ein typisches erfindungsgemäßes Implantat 10 eine erste Schnittstelle 30 und eine zweite Schnittstelle 32 aufweist, die beide mit einer Steuereinheit 34 und einem Speicher 36 verbunden sind. Die erste Schnittstelle 30 dient dabei der bidirektionalen drahtlosen Kommunikation mit einem Programmiergerät, und die zweite Schnittstelle 32 dient der bidirektionalen Datenkommunikation mit einem externen Gerät bzw. Patientengerät als Relaisstation für eine bidirektionale Datenkommunikation mit einem zentralen Servicecenter. In dem Speicher 36 können vom Implantat 10 selbst generierte Daten oder seitens des Programmiergerätes oder des zentralen Servicecenters empfangene Daten gespeichert werden. Die Steuereinheit 34 ist ausgebildet, seitens des zentralen Servicecenters generierte und an ein Programmiergerät gerichtete Datenpakete zu erkennen und nötigenfalls in dem Speicher 36 zwischenzuspeichern, um sie im Falle einer bidirektionalen Datenkommunikation mit einem Programmiergerät an jenes weiterzuleiten.

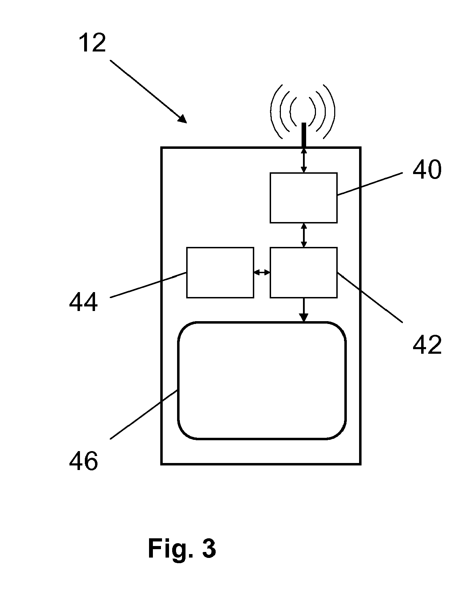

Das in Fig. 3 abgebildete erfindungsgemäße Programmiergerät 12 besitzt eine Schnittstelle 40 für die bidirektionale drahtlose Datenübertragung zwischen einem Implantat 10 und dem Programmiergerät 12. Diese Schnittstelle 40 des Programmiergerätes 12 ist mit einer Steuereinheit 42 des Programmiergerätes 12 sowie einem Speicher 44 des Programmiergerätes 12 verbunden. Die Steuereinheit 42 des Programmiergerätes 12 ist außerdem mit einer Anzeige 46 des Programmiergerätes 12 verbunden. Die Anzeige 46 kann beispielsweise der Anzeige von Textnachrichten oder auch der Anzeige von intrakardial gewonnenen Elektrokardiogrammen in grafischer Darstellung dienen. Solche intrakardialen Elektrokardiogramme werden beispielsweise in einem Implantat 10 generiert und bei der Abfrage des Implantats 10 durch das Programmiergerät 12 von dem Implantat 10 zum Programmiergerät 12 übertragen.

Erfindungsgemäß ist die Steuereinheit 42 des Programmiergerätes 12 zusätzlich dazu ausgebildet, solche seitens des Implantats 10 empfangenen Datenpakete zu erkennen, die von einem zentralen Servicecenter generiert wurden und die Textnachrichten oder Steuer- und Programmierbefehle für das Programmiergerät 12 enthalten. Solche Datenpakete werden seitens der Steuerung 42 des Programmiergerätes decodiert und führen anschließend beispielsweise zu einer Anzeige einer Textnachricht auf der Anzeige 46. Im Falle von Steuer- oder Programmierbefehlen kann der Empfang derselben die Steuerung 42 des Programmiergerätes 12 auch veranlassen, beispielsweise bestimmte Abfragen des Implantats vorzunehmen oder auch ein Firmware-Update des Programmiergerätes 12 selbst.

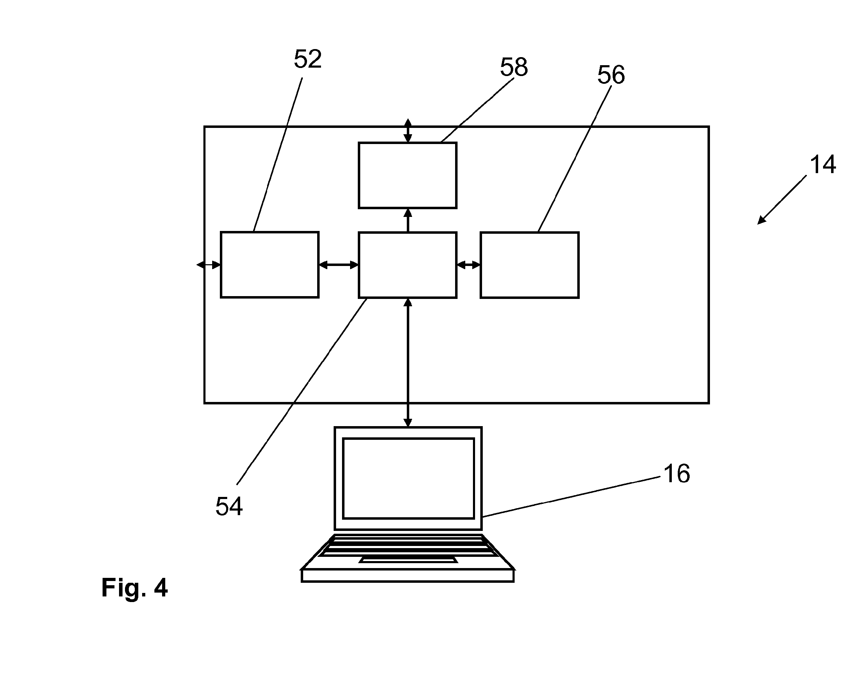

Das in Fig. 4 abgebildete erfindungsgemäße Servicecenter 14 besitzt neben einer weiteren Schnittstelle 52 für die bidirektionale Datenübertragung zwischen dem Server 14 und einem Implantat 10 eine Steuereinheit 54, die dazu ausgebildet ist, Datenpakete zu generieren, die an ein mit dem Implantat 10 gelegentlich zu verbindendes Programmiergerät 12 gerichtet sind. Dazu ist die Steuereinheit 54 des Servers 14 beispielsweise mit einem Terminal 16 zur Eingabe solcher Textnachrichten verbunden oder mit einer automatischen Auswerteeinheit 56 für die Auswertung von seitens des Implantats empfangenen Daten, beispielsweise physiologischen Daten eines Patienten oder Betriebsdaten eines Implantats 10. Weiterhin besitzt der Server 14 vorzugsweise eine weitere Schnittstelle 58, über die ein Hersteller 26 eines Implantats 10 Zugriff auf das zentrale Servicecenter 14 hat.

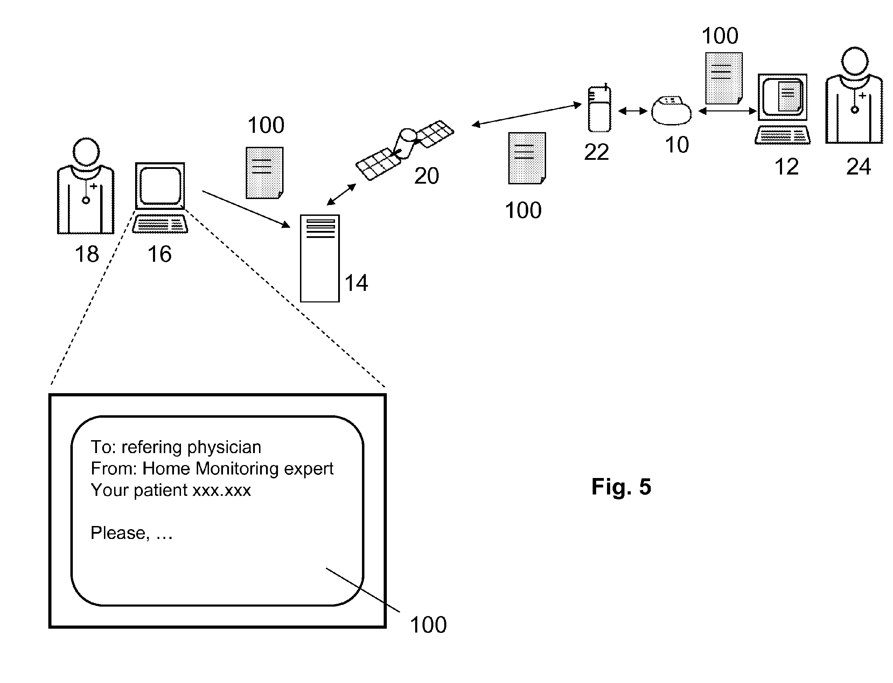

Fig. 5 zeigt ein Beispiel, wie seitens des Implantats 10 an den zentralen Server 14 übersandte Daten von einem spezialisierten Arzt 18 auf dessen Terminal 16 ausgewertet werden. Dieser Arzt 18 hat dann die Möglichkeit, auf seinem Terminal 16 eine Text- oder Sprachnachricht 100 für einen nachsorgenden Arzt 24, der u.U. weit weniger spezialisiert ist, zu verfassen und an den Server 14 zu senden. Der Server 14 sendet dann die implantatspezifische Nachricht 100 automatisch über die Kommunikationsverbindung 20 an das Patientengerät 22. Dieses überträgt dann die empfangene Information 100 an das elektronische Implantat 10 bei der nächstmöglichen Verbindung zwischen elektronischem Implantat 10 und Patientengerät 22.

Bei einer regulären Nachsorge dieses elektronischen Implantats 10 durch den entsprechenden Nachsorgearzt 24 wird dann die im Implantat 10 in einem eigens dafür reservierten Speicherbereich des Speichers 36 abgelegte Nachricht 100 durch das Programmiergerät 12 abgefragt und auf dessen Anzeige angezeigt.

Das Implantat 10 fungiert somit quasi als Mailbox für das Patientengerät 12.

Optional kann das Programmiergerät 12 automatisch oder manuell durch den nachsorgenden Arzt 24 den Erhalt eines Datenpakets oder einer Nachricht 100 quittieren und diese Information in Form eines vom Programmiergerät 12 generierten Datenpakets 100 an das Implantat 10 übertragen, wo es in dessen Speicher 36 gespeichert wird. Diese Empfangsquittung wird dann über das Patientengerät 22 und über die Kommunikationsverbindung 20 an den Server 14 im Falle der nächsten regulären Datenkommunikation zwischen Implantat und Servicecenter gesendet. Die so übertragene Empfangsquittung kann dann dem fernüberwachenden Arzt auf dessen Terminal angezeigt werden.

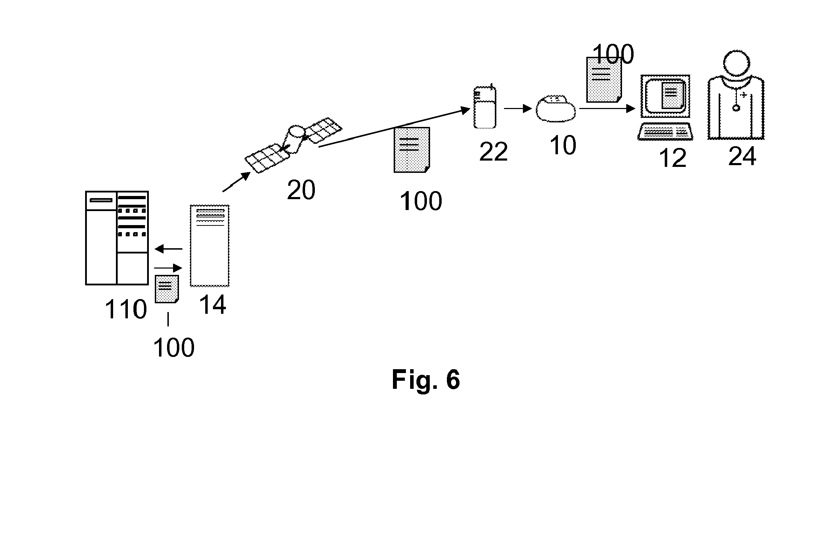

In Fig. 6 ist die Information eines nachsorgenden Arztes 24 durch eine automatische Analyse von seitens eines Implantats 10 empfangenen Daten im Server 14 dargestellt. Dabei werden die im Server 14 gespeicherten Daten eines elektronischen Implantates 10 mit einer Auswertesoftware in einem Analyserechner 110 automatisch ausgewertet. Der Analyserechner 110 generiert bei Erfüllung entsprechender Kriterien automatisch einen "Ereignisreport" 100. Dieser Ereignisreport 100 wird in Form eines automatisch generierten Datenpakets 100 von dem Analyserechner 110 an den Server 14 versendet und dann über die Kommunikationsverbindung 20 und Patientengerät 22 über den zweiten Datenkommunikationskanal an das betreffende Implantat 10 zugestellt und dort gespeichert.

Im Falle der Nachsorge durch einen nachsorgenden Arzt 24 kann dieser den im Implantat 10 abgelegten Ereignisreport 100 mit einem Programmiergerät 12 abfragen und über das Programmiergerät 12 einsehen.

Auf diese Weise ist es möglich, weit komplexere automatische Auswertealgorithmen zur Analyse von seitens des elektronischen Implantats 10 generierten Daten anzuwenden, als dies in einem Implantat 10 oder in einem Programmiergerät 12 derzeit möglich wäre. Insbesondere können so sehr viel mehr Trenddaten, also beispielsweise über mehrere Jahre gesammelte Daten eines oder mehrerer Parameter, verarbeitet werden, da die Datenspeicherung im Server 14 und nicht im elektronischen Implantat 10 erfolgt. Ebenso ist die Rechenleistung eines typischen Analyserechners 110 weit größer als die eines Programmiergerätes 12 oder gar eines Implantats 10.

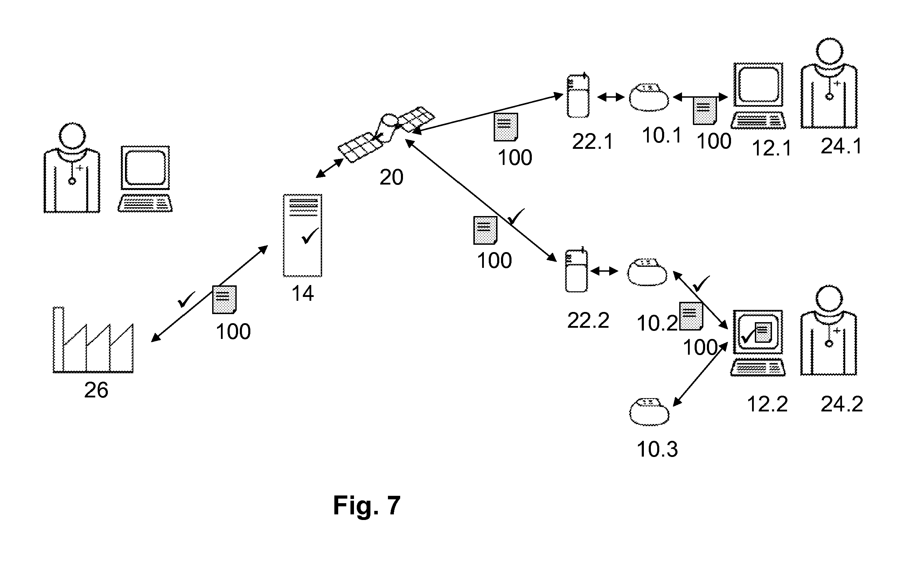

In Fig. 7 ist die Anwendung des in Fig. 1 dargestellten Systems zur Verteilung einer Herstellerinformation 100 an einen nachsorgenden Arzt 24 dargestellt. Eine solche Herstellerinformation 100, beispielsweise eine "Product Safety Advisory", wird von Hersteller 26 eines Implantats 10 in den Server 14 derart eingestellt, dass diese Information an alle elektronischen Implantate 10.1 und 10.2, die mit dem Server 14 verbunden sind, gesendet wird. Bei der Nachsorge eines solchen Implantats 10.1 bzw. 10.2 wird diese Herstellerinformation 100 durch das Programmiergerät 12 ausgelesen und auf dem Programmiergerät 12 gespeichert.

Angezeigt wird diese Herstellerinformation 100 auf der Anzeige 46 des Programmiergerätes 12 jedoch nur dann, wenn ein Implantat 10.1 bzw. 10.2, das von dieser Herstellerinformation 100 betroffen ist, nachgesorgt wird. Mit einem Programmiergerät 12 können nämlich jeweils verschiedene Implantate 10 nachgesorgt werden.

Auf diese Weise ist es auch möglich, Herstellerinformationen 100 oder andere Informationen für Implantate 10.3 auf dem Programmiergerät 12.2 anzuzeigen, die selbst nicht über eine Schnittstelle für eine Datenkommunikation mit einem zentralen Servicecenter 14 verfügen. Dies ist im Beispiel des Implantats 10.3 dargestellt. Die Herstellerinformation 100 in Fig. 7 bezieht sich nur auf dieses Implantat 10.3 und wird daher auch nur dann angezeigt, wenn das Implantat 10.3 nachgesorgt wird, jedoch nicht ein anderes Implantat 10.1 oder 10.2.

Optional kann das Programmiergerät 12.2 mittels seiner Steuereinheit 42 dazu ausgebildet sein, die Nachsorge eines Implantats 10.3 nebst Anzeige der Herstellerinformation 100 zu quittieren, indem die Steuereinheit 42 ein entsprechendes Quittungs-Datenpaket 100 generiert und dieses Quittungs-Datenpaket 100 über das nächste Implantat 10.2, mit dem das Programmiergerät 12.2 anschließend verbunden wird und das eine Schnittstelle 32 zur bidirektionalen Datenkommunikation mit dem zentralen Servicecenter besitzt, zurück an den Server 14 sendet. Dazu wird zunächst das Quittungs-Datenpaket 100, welches die Nachsorge eines Implantats 10.3 bestätigt, im Programmiergerät 12 gespeichert. Anschließend wird dieses Quittungs-Datenpaket 100 vom Programmiergerät 12 an ein Implantat 10.2 mit einer Schnittstelle 32 für die bidirektionale Datenkommunikation mit dem zentralen Servicecenter übertragen. Dieses Implantat 10.2 sendet das Quittungsdatenpaket 100 mit einer nächsten Nachricht über das entsprechende Patientengerät 22.2 und den Satelliten 20 an den Server 14. Der Hersteller 26 des Implantats 10.3 kann auf diese Weise eine Nachsorgestatistik für die betroffenen Implantate 10 vom Server 14 abfragen. Diese Nachsorgestatistik kann dabei sowohl solche Implantate 10.1 und 10.2 enthalten, die über eine Schnittstelle 32 für die bidirektionale Datenkommunikation zu einem zentralen Servicecenter verfügen, als auch solche Implantate 10.3, die nicht über eine solche Schnittstelle 32, sondern nur über eine Schnittstelle 30 für die Datenkommunikation mit einem Programmiergerät 12.

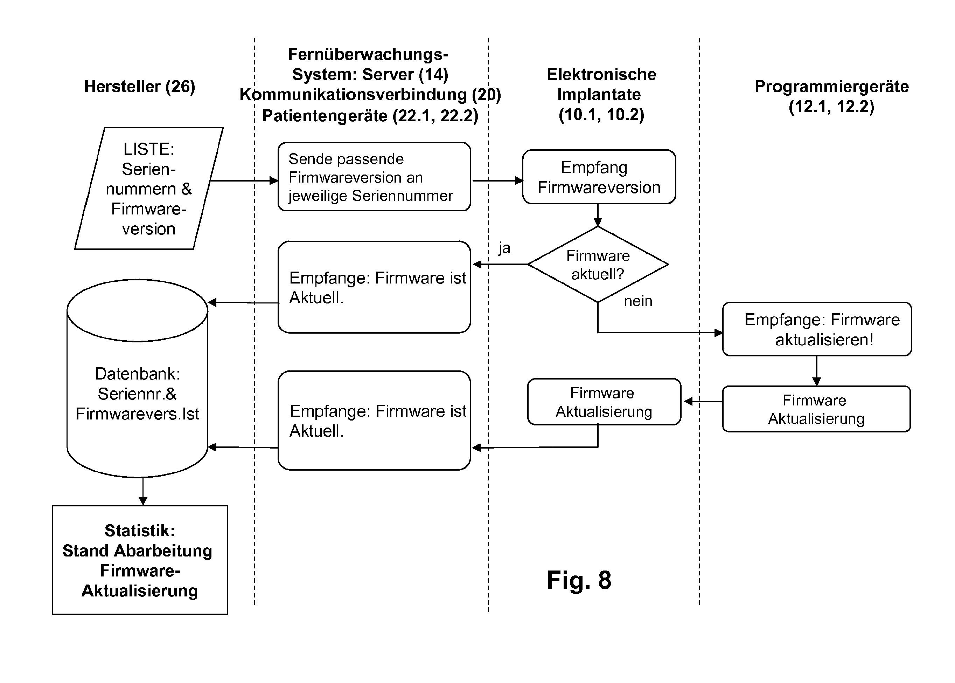

In Fig. 8 ist der Ablauf für den Fall eines Firmware-Updates einzelner elektronischer Implantate 10 dargestellt.

Der Hersteller 26 elektronischer Implantate 10 hinterlegt im Server 14 eine Liste von Seriennummern und zugehörigen Firmware-Versionsnummern. Diese Information wird dann vom Server 14 über über die Kommunikationsverbindung 20 und das Patientengerät 22 zu einem jeweils betroffenen elektronischen Implantat 10 übertragen, so dass in dem jeweiligen elektronischen Implantat 10 eine Information zwischengespeichert wird, welche Firmware für dieses Implantat vom Hersteller gefordert wird. Alternativ kann die gesamte Seriennummernliste nebst Firmware-Information an alle elektronischen Implantate 10 gesendet und dort gespeichert werden.

Das jeweilige elektronische Implantat 10 vergleicht die gewünschte mit der aktuellen Firmware-Version. Stimmen diese überein, so wird an den Server 14 eine Bestätigung gesendet, dass die Firmware des jeweiligen Implantats 10 mit der gewünschten Firmware übereinstimmt.

Stimmt die Firmware-Version nicht überein, so wird bei der nächsten regulären Nachsorge die Information über die gewünschte Firmware-Version vom Programmiergerät 12 ausgelesen und, basierend auf dieser Information, der Firmware-Update-Prozess gestartet. Ist die Firmware entsprechend aktualisiert, sendet das elektronische Implantat 10 mit der nächsten periodischen Nachricht eine entsprechende Bestätigung an den Server 14.

Die Bestätigungen des erfolgreichen Firmware-Updates werden dann vom Hersteller 26 im Server 14 abgefragt und in einer entsprechenden Datenbank gespeichert. So können sie statistisch ausgewertet werden.

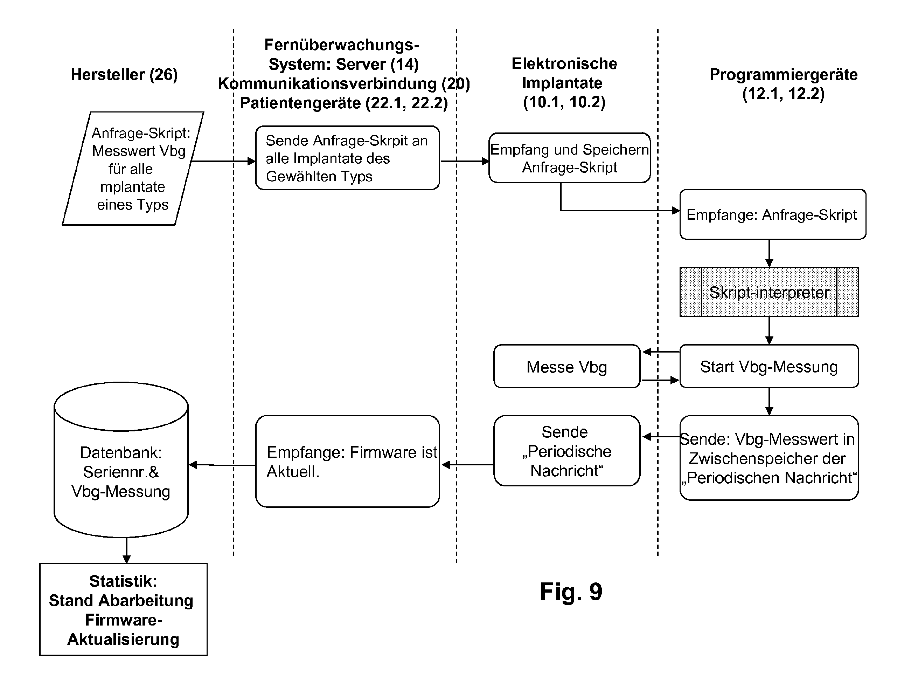

Fig. 9 zeigt nun den Ablauf der Steuerung einer Programmiergerätkomponente. In diesem Fall wird vom Hersteller 26 eines Implantats 10 eine Anfrage an alle Implantate 10 eines bestimmten Typs gestellt, beispielsweise einen spezifischen Messwert, wie die Bandgap-Spannung Vbg, zur Produktüberwachung abzufragen. Der Hersteller 26 sendet über den Server 14, einen Satelliten 20 und ein Patientengerät 22 ein Datenpaket mit einem interpretierbaren Skript an alle betroffenen elektronischen Implantate 10. Dieses Skript wird im jeweiligen Implantat 10 gespeichert und bei dessen Nachsorge vom Programmiergerät 12 ausgelesen. Die Programmiergeräte-Software (d.h. die Steuereinheit 42 des Programmiergeräts 12) ist in diesem Ausführungsbeispiel um einen entsprechenden Skript-Interpreter erweitert. Dieser Skript-Interpreter setzt das übertragene Skript zur Bandgap-Spannungsmessung in Programmiergerät-Kommunikationskommandos um, die eine Bandgap-Messung im elektronischen Implantat 10 im Rahmen der regulären Nachsorge bewirken. Der entsprechende Messwert wird nach Abschluss der Messung vom Programmiergerät 12 ausgelesen und anschließend in den Datentransferspeicher 36 eines Implantats 10 eingetragen, so dass dieser Bandgap-Spannungsmesswert mit der nächsten regulären Datenübertragung vom elektronischen Implantat 10 zum Server 14 ermittelt wird. Der Hersteller 26 des Implantats 10 kann diese Messwerte am Server 14 abfragen und in einer entsprechenden Datenbank statistisch auswerten.

## Patentansprüche

1. Medizinisches Implantat (10) mit wenigstens einer Schnittstelle (30, 32) zum bidirektionalen drahtlosen Datenaustausch, einem Datenspeicher (36) und einer mit der Schnittstelle (30, 32) und dem Datenspeicher (36) verbundenen Steuerung (34), wobei die Steuerung (34) in Verbindung mit der Schnittstelle (30, 32) ausgebildet ist, einen drahtlosen Datenaustausch mit wenigstens zwei unterschiedlichen externen Geräten (12, 22) durchzuführen, so dass sich wenigstens zwei verschiedene Datenkommunikationskanäle ergeben, von denen ein erster der Datenkommunikation mit einem Programmiergerät (12) zugeordnet ist und ein zweiter Datenkommunikationskanal der Datenkommunikation mit einem zentralen Servicecenter (14) zugeordnet ist,
**dadurch gekennzeichnet, dass** das medizinische Implantat (10) weiter ausgebildet ist, über den zweiten Datenkommunikationskanal Steuerbefehle und/oder Nachrichten zu empfangen, während der zweite Datenkommunikationskanal aktiv ist, empfangene Steuerbefehle und/oder Nachrichten, die für ein Programmiergerät (12) bestimmt sind, zu erkennen, erforderlichenfalls in dem Speicher (36) zwischenzuspeichern und über den ersten Datenkommunikationskanal an ein Programmiergerät (12) weiterzuleiten, während der erste Datenkommunikationskanal aktiv ist.

2. Medizinisches Implantat (10) nach Anspruch 1, **dadurch gekennzeichnet, dass** das medizinische Implantat (10) zwei Schnittstellen (30, 32) aufweist, von denen eine erste Schnittstelle (30) dem ersten Datenkommunikationskanal für einen bidirektionalen, drahtlosen Datenaustausch mit einem Programmiergerät (12) zugeordnet ist und die zweite Schnittstelle (32) dem zweiten Datenkommunikationskanal für einen bidirektionalen, drahtlosen Datenaustausch mit einem zentralen Servicecenter (14) zugeordnet ist.

3. Programmiergerät (12) zur drahtlosen Datenabfrage und Programmierung eines medizinischen Implantats (10), mit einer Schnittstelle (40) zum bidirektionalen drahtlosen Datenaustausch mit einem medizinischen Implantat (10), einem Datenspeicher (44), einer Anzeige (46) und einer mit der Schnittstelle (40), der Anzeige (46) und dem Datenspeicher (44) verbundenen Steuerung (42), wobei die Steuerung (42) ausgebildet, über die Schnittstelle (40) empfangene Datenpakete (100) wenigstens derart zu unterscheiden und zu verarbeiten, dass Datenpakete (100), die eine Textnachricht enthalten zum Anzeigen der Textnachricht auf der Anzeige führen und Datenpakete (100), die Steuer- oder Programmierbefehle enthalten, zum Ausführen dieser Steuer- oder Programmierbefehle.

4. Programmiergerät (12) nach Anspruch 3, **dadurch gekennzeichnet, dass** das Programmiergerät (12) ausgebildet ist, nach Empfang eines von einem zentralen Servicecenter (14) generierten Datenpakets (100) eine an das jeweilige zentrale Servicecenter (14) gerichtetes Quittungs-Datenpaket mit einer Empfangsbestätigung zu generieren.

5. Medizinisches Therapiesystem mit einem Implantat (10) nach Anspruch 1 oder 2 und einem zentralen Servicecenter (14), welches wenigstens zeitweise über einen Datenkommunikationskanal mit dem medizinischen Implantat (10) zum Datenaustausch verbunden ist, **dadurch gekennzeichnet, dass** das zentrale Servicecenter (14) ausgebildet ist, an ein mit dem medizinischen Implantat (10) zu verbindendes Programmiergerät (12) gerichtete Datenpakete (100) mit Textnachrichten und/oder Steuer- und Programmierbefehlen zu generieren und an das medizinische Implantat (10) zu übertragen, wenn der Datenkommunikationskanal aktiv ist.

6. Zentrales Servicecenter (14) für ein medizinisches Therapiesystem nach Anspruch 5, **dadurch gekennzeichnet, dass** das zentrale Servicecenter (14) ausgebildet ist, an ein mit dem medizinischen Implantat (10) zu verbindendes Programmiergerät (12) gerichtete Datenpakete (100) mit Textnachrichten und/oder Steuer- und Programmierbefehlen zu generieren.

7. Zentrales Servicecenter (14) nach Anspruch 6, **dadurch gekennzeichnet, dass** das zentrale Servicecenter (14) eine Schnittstelle zur Eingabe von Textnachrichten, die für ein Programmiergerät (12) bestimmt sind, aufweist.

8. Verfahren zur Übertragung von Daten von einem zentralen Servicecenter zu einem Programmiergerät für ein medizinisches Implantat, **dadurch gekennzeichnet, dass** das zentrale Servicecenter ein an ein Programmiergerät gerichtetes Datenpaket generiert und über einen zweiten Datenkommunikationskanal an ein medizinisches Implantat überträgt, welches das Datenpaket entweder zwischenspeichert oder unmittelbar über einen ersten Datenkommunikationskanal an ein Programmiergerät überträgt.

9. Verfahren nach Anspruch 8, **dadurch gekennzeichnet, dass** das vom zentralen Servicecenter generierte Datenpaket eine Textnachricht enthält und dass das Programmiergerät diese Textnachricht nach Empfang des Datenpaketes automatisch auf einer Anzeige anzeigt.

10. Verfahren nach Anspruch 8 oder 9, **dadurch gekennzeichnet, dass** das vom zentralen Servicecenter generierte Datenpaket Programmier- oder Steuerbefehle enthält und dass das Programmiergerät diese Programmier- oder Steuerbefehle nach Empfang des Datenpaketes automatisch ausführt.
